(19) Europäisches Patentamt / European Patent Office / Office européen des brevets

(11) EP 2 947 088 B1

(12) EUROPÄISCHE PATENTSCHRIFT

(45) Veröffentlichungstag und Bekanntmachung des Hinweises auf die Patenterteilung:
**07.11.2018 Patentblatt 2018/45**

(51) Int Cl.:
***C07F 9/145*** *(2006.01)* ***B01J 31/18*** *(2006.01)*
***C07C 45/50*** *(2006.01)*

(21) Anmeldenummer: **15166048.7**

(22) Anmeldetag: **30.04.2015**

# (54) VERFAHREN ZUR KATALYTISCHEN HERSTELLUNG VON ALDEHYDEN AUS OLEFINEN UNTER EINSATZ VON MONOPHOSPHIT-GEMISCHEN

METHOD FOR THE CATALYTIC PREPARATION OF ALDEHYDES FROM OLEFINS USING MONOPHOSPHITE MIXTURES

PROCÉDÉ DE PRODUCTION CATALYTIQUE D'ALDÉHYDES À PARTIR D'OLÉFINES À L'AIDE DE MÉLANGES DE MONOPHOSPHITES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.05.2014 DE 102014209535**
**16.02.2015 DE 102015202722**

(43) Veröffentlichungstag der Anmeldung:
**25.11.2015 Patentblatt 2015/48**

(73) Patentinhaber: **Evonik Degussa GmbH**
**45128 Essen (DE)**

(72) Erfinder:
- **Dyballa, Katrin Marie**
  **45657 Recklinghausen (DE)**
- **Franke, Robert**
  **45772 Marl (DE)**
- **Fridag, Dirk**
  **45721 Haltern am See (DE)**
- **Hess, Dieter**
  **45770 Marl (DE)**
- **Hamers, Bart**
  **5961 VG Horst (NL)**
- **Geilen, Frank**
  **45721 Haltern am See (DE)**

(56) Entgegenhaltungen:
**WO-A1-02/070625 WO-A1-2010/151285**
**WO-A1-2014/056737 DE-T2- 69 707 145**
**US-A- 5 672 766 US-A1- 2011 196 079**

- **MANFRED T. REETZ ET AL: "The Influence of Mixtures of Monodentate Achiral Ligands on the Regioselectivity of Transition-Metal-Catalyzed Hydroformylation", ANGEWANDTE CHEMIE INTERNATIONAL EDITION, Bd. 44, Nr. 19, 6. Mai 2005 (2005-05-06), Seiten 2962-2964, XP055202904, ISSN: 1433-7851, DOI: 10.1002/anie.200462613**
- **ROBERT FRANKE ET AL: "Applied Hydroformylation", CHEMICAL REVIEWS, Bd. 112, Nr. 11, 14. November 2012 (2012-11-14), Seiten 5675-5732, XP055091930, ISSN: 0009-2665, DOI: 10.1021/cr3001803**
- **Anonymous: "DOVERPHOS® S-9228 & DOVERPHOS® S-9411 HIGH PERFORMANCE PHOSPHITE STABILIZERS", Dover Chemical Corporation , 2011, XP002742356, Gefunden im Internet: URL:http://www.doverchem.com/Portals/0/DP9228-9411.pdf [gefunden am 2015-07-16]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 947 088 B1

## Beschreibung

Die Erfindung betrifft Verfahren zur katalytischen Herstellung von Aldehyden aus Olefinen unter Einsatz von Monophosphit-Gemischen, sowie die neuen Monophosphit-Gemischen selbst.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor $P^{III}$. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Die Art des Katalysatorsystems und die optimalen Reaktionsbedingungen für die Hydroformylierung sind von der Reaktivität des eingesetzten Olefins abhängig.

Die unterschiedliche Reaktivität von isomeren Octenen ist ebenfalls bekannt (siehe B. L. Haymore, A. van Hasselt, R. Beck, Annals of the New York Acad. Sci., 415, 1983, S.159-175).

Über die unterschiedlichen Prozesse und Katalysatoren sind eine Vielzahl von Olefinen für die Hydroformylierung zugänglich (siehe P. W. N. M. van Leeuwen, in Rhodium Catalyzed Hydroformylation, P. W. N. M. van Leeuwen, C. Claver (Hrsg.), Kluwer, Dordrecht, 2000). Technische Olefingemische, die als Edukte für die Oxo-Synthese verwendet werden, enthalten oftmals Olefine der verschiedensten Strukturen mit unterschiedlichen Verzweigungsgraden, unterschiedlicher Lage der Doppelbindung im Molekül und ggf. auch mit einer unterschiedlichen Kohlenstoffanzahl. Besonders gilt dies für Olefingemische, die durch Di-, Tri- oder weitgehende Oligomerisierung von Olefinen entstanden sind. Als Beispiele für technische Olefingemische, die durch Hydroformylierung zu den entsprechenden Aldehydgemischen umgesetzt werden, seien Tri- und Tetrapropen sowie Di-, Tri- und Tetrabuten genannt.

Die oben genannten technischen Olefingemische enthalten oftmals nur geringe Anteile an Olefinen mit endständigen Doppelbindungen. Um hieraus Produkte herzustellen, in denen mehr terminal hydroformyliertes Aldehyd vorliegt als im ursprünglichen Olefingemisch, muss unter isomerisierenden Bedingungen hydroformyliert werden.

Geeignete Verfahren dafür sind beispielsweise Hochdruck-Hydroformylierungen mit Cobaltkatalysatoren. Diese Verfahren haben aber u.a. den Nachteil, dass relativ viele Nebenprodukte wie Alkane, Acetale und Ether gebildet werden und sehr drastische Reaktionsbedingungen (hohe Temperatur, hoher Druck) notwendig sind (siehe auch Klaus-Diether Wiese, Dietmar Obst, Top. Organomet. Chem. 2006, 18, 1-33).

Bei der Verwendung von Rhodiumkomplexen als Katalysator ist der Ligand für die Produktzusammensetzung der Aldehyde mitbestimmend. Nichtmodifizierte Rhodiumcarbonylkomplexe katalysieren die Hydroformylierung von Olefinen mit end- und innenständigen Doppelbindungen, wobei die Olefine auch verzweigt sein können, zu Aldehyden mit einem hohen Verzweigungsgrad. Der Anteil an terminal hydroformyliertem Olefin ist im Vergleich zum Cobalt-katalysierten Produkt deutlich geringer.

Die Hydroformylierung von Olefinen mit innenständigen Doppelbindungen an Katalysatorsystemen, die sterisch anspruchsvolle Bisphosphitliganden enthalten, erfolgt bei langkettigen Olefinen zwar mit guter Selektivität, jedoch nicht mit einer zufriedenstellenden Aktivität (P. W. N. M. van Leeuwen, in Rhodium Catalyzed Hydroformylation, P. W. N. M. van Leeuwen, C. Claver (Hrsg.), Kluwer, Dordrecht, 2000).

In Angew. Chem. Int. Ed. 2000, 39, No. 9, S.1639-1641 von Börner et al. werden Phosphonite in der Hydroformylierung eingesetzt, also Liganden, welche eine P-C- und zwei P-O-Bindungen aufweisen. Die hier beschriebenen Phosphonite weisen bei einem Einsatz in der Hydroformylierung n/iso-Selektivität (n/iso = das Verhältnis von linearem Aldehyd (= n) zu verzweigtem (= iso) Aldehyd)) von 0,61 bis 1,57 auf.

Allerdings ist die Herstellung dieser Liganden auf Basis einer Phosphonitstruktur im Falle einer großtechnischen Synthese deutlich aufwändiger als beispielsweise die Herstellung von Phosphitliganden. Dieser Punkt spielt insbesondere bei dem Einsatz dieser Liganden in einem großtechnischen Prozess eine elementare Rolle. Die Synthese der als Liganden eingesetzten Verbindungen sollte so günstig und einfach wie möglich sein.

Rhodium-Monophosphit-Komplexe in katalytisch aktiven Zusammensetzungen hingegen sind geeignet für die Hydroformylierung von verzweigten Olefinen mit innenständigen Doppelbindungen.

Seit den 1970er Jahren ist die Verwendung von so genannten "bulky phosphites" in der Hydroformylierung beschrieben (siehe u.a. van Leeuwen et. al, Journal of Catalysis, 2013, 298, 198-205). Diese zeichnen sich durch eine gute Aktivität aus, jedoch ist die n/i-Selektivität für endständig hydroformylierte Verbindungen verbesserungswürdig.

Neben dem Einsatz von reinen Liganden ist auch der Einsatz von Ligandenmischungen in der Literatur beschrieben.

In US 20120253080 wird der Einsatz von Monophosphiten mit Bisphosphiten beschrieben. Jedoch hat diese Kombination den Nachteil, dass die Bisphosphite zwar eine gute Selektivität besitzen, im Fall von langkettigen Olefinen ist ihre Aktivität jedoch sehr gering und somit verbesserungswürdig. In einem großtechnischen Prozess ist neben der

Selektivität zum gewünschten Produkt auch die Raum-Zeit-Ausbeute bzw. die Aktivität des Katalysatorsystems ein wichtiger Faktor im Hinblick auf dessen Rentabilität. Außerdem sind die Bisphosphite häufig in ihrer Herstellung deutlich teurer als beispielsweise die Monophosphite.

In EP 1 099 678 wird der Einsatz von Phosphoniten mit Bisphosphiten beschrieben. Hierbei ist jedoch nachteilig, dass beide Ligandentypen in ihrer Herstellung sehr teuer sind, und ein großtechnischer Prozess somit kaum wirtschaftlich sein kann. Außerdem beeinflusst der Zusatz des Bisphosphitliganden die Ausbeute der Reaktion merklich, da diese Liganden bei der Verwendung von z.B. Dibuten als Substrat weniger aktiv sind.

In WO 2007/149143 werden Mischungen von zwei bis fünf Triarylphosphiten offenbart, die unter Umgebungsbedingungen flüssig sind. Diese Triarylphosphite weisen mindestens einen AlkylSubstituenten, genauer einen tert.-Butyl- oder tert.-Pentyl-Substituenten auf. Die Mischungen werden als Stabilisatoren/Antioxidantien für Polymerharze, insbesondere thermoplastische Harze oder Elastomere verwendet.

US 8,258,215 B2 beschreibt Phosphite, welche zur Stabilisierung von Polymeren eingesetzt werden. Diese Phosphite weisen entweder einen aromatischen und zwei aliphatische Reste auf, oder zwei aromatische und einen aliphatischen Rest.

In MANFRED T. REETZ ET AL, "The Influence of Mixtures of Monodentate Achiral Ligands on the Regioselectivity of Transition-Metal-Catalyzed Hydroformylation", ANGEWANDTE CHEMIE INTERNATIONAL EDITION, (20050506), Bd. 44, Nr. 19, Seiten 2962 - 2964 wird der Einfluß von Mischungen monodentater Liganden auf die Regioselektivität bei der Hydroformylierung untersucht.

Es ist daher wünschenswert, ein Katalysatorsystem zu entwickeln, das die im Stand der Technik gezeigten Nachteile nicht aufweist.

Aufgabe der vorliegende Erfindung war es daher, ein Katalysatorsystem zur Hydroformylierung von Olefinen bereitzustellen, mit dem verzweigte, unverzweigte, end- und innenständige Olefine mit hohen Ausbeuten und Selektivitäten terminal hydroformyliert werden könne, d.h. möglichst lineare Aldehyde hergestellt werden können.
Des Weiteren soll das Verhältnis Kosten / Nutzen der zum Einsatz kommenden Liganden optimiert werden.

Die Aufgabe wird gelöst durch ein Gemisch nach Anspruch 1.

Gemisch umfassend eine Verbindungen gemäß der Strukturen **IIIb:**

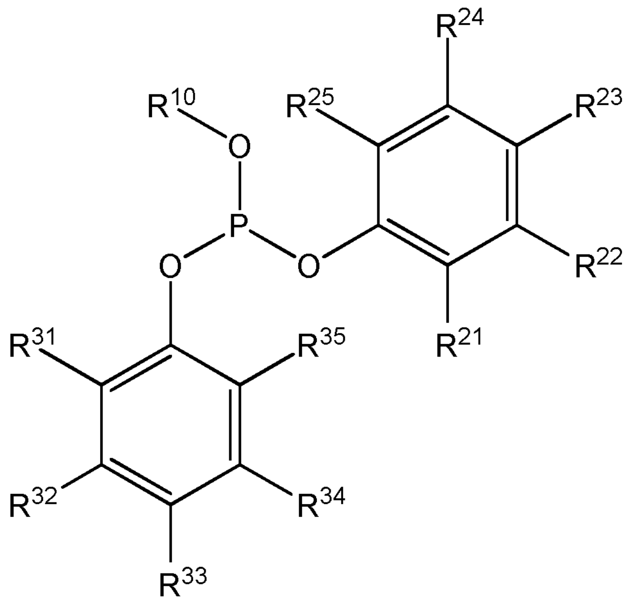

**IIIb**

wobei

$R^{10}$ ausgewählt ist aus:

-($C_1$-$C_{12}$)-Alkyl,

$R^{21}$, $R^{22}$, $R^{23}$, $R^{24}$, $R^{25}$, $R^{31}$, $R^{32}$, $R^{33}$, $R^{34}$, $R^{35}$ jeweils unabhängig voneinander ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CO-($C_1$-$C_{12}$)-Alkyl, -CO-($C_6$-$C_{20}$)-Aryl, -COOH, -OH;
sowie

umfassend eine Verbindung gemäß der Struktur **IIb:**

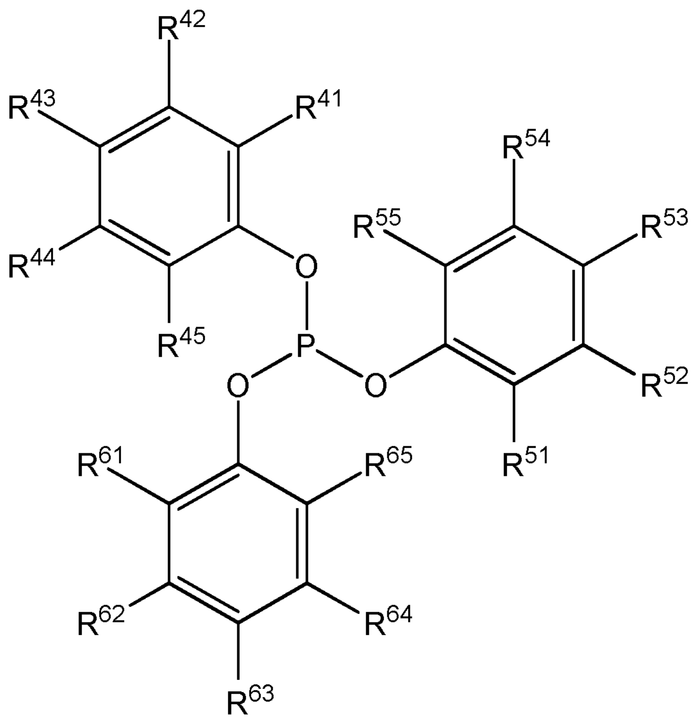

**IIb**

wobei

$R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ jeweils unabhängig voneinander ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CO-($C_1$-$C_{12}$)-Alkyl, -CO-($C_6$-$C_{20}$)-Aryl, -COOH, -OH.

($C_1$-$C_{12}$)-Alkyl kann jeweils unsubstituiert oder durch einen oder mehrere gleiche oder verschiedene Reste substituiert sein, die ausgewählt sind unter ($C_3$-$C_{12}$)-Cycloalkyl, ($C_3$-$C_{12}$)-Heterocycloalkyl, ($C_6$-$C_{20}$)-Aryl, Fluor, Chlor, Cyano, Formyl, Acyl oder Alkoxycarbonyl.

($C_6$-$C_{20}$)-Aryl kann jeweils unsubstituiert oder durch einen oder mehrere gleiche oder verschiedene Reste substituiert sein, die ausgewählt sind unter -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen (wie Cl, F, Br, I), -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, - COOH, -OH, -$SO_3H$; -$SO_3Na$, -$NO_2$, -CN, -$NH_2$, -N[($C_1$-$C_{12}$)-Alkyl]$_2$.

($C_3$-$C_{12}$)-Cycloalkyl kann jeweils unsubstituiert oder durch einen oder mehrere gleiche oder verschiedene Reste substituiert sein, die ausgewählt sind unter ($C_1$-$C_{12}$)-Alkyl, ($C_1$-$C_{12}$)-Alkoxy, ($C_3$-$C_{12}$)-Cycloalkyl, ($C_3$-$C_{12}$)-Heterocycloalkyl, ($C_6$-$C_{20}$)-Aryl, Fluor, Chlor, Cyano, Formyl, Acyl oder Alkoxycarbonyl.

Im Rahmen der Erfindung umfasst der Ausdruck -($C_1$-$C_{12}$)-Alkyl geradkettige und verzweigte Alkylgruppen. Vorzugsweise handelt es sich dabei um unsubstituierte geradkettige oder verzweigte -($C_1$-$C_8$)-Alkyl- und ganz bevorzugt -($C_1$-$C_6$)-Alkylgruppen. Beispiele für -($C_1$-$C_{12}$)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Substituierte -($C_1$-$C_{12}$)-Alkylgruppen können in Abhängigkeit von ihrer Kettenlänge, einen oder mehrere Substituenten aufweisen. Die Substituenten sind vorzugsweise unabhängig voneinander ausgewählt unter -($C_3$-$C_{12}$)-Cycloalkyl, -($C_3$-$C_{12}$)-Heterocycloalkyl, -($C_6$-$C_{20}$)-Aryl, Fluor, Chlor, Cyano, Formyl, Acyl oder Alkoxycarbonyl.

Der Ausdruck -($C_3$-$C_{12}$)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Substituierte -($C_3$-$C_{12}$)-Cycloalkylgruppen können, in Abhängigkeit von ihrer Ringgröße, einen oder mehrere (z.B. 1, 2, 3, 4 oder 5) weitere Substituenten aufweisen. Diese Substituenten sind vorzugsweise unabhängig voneinander ausgewählt unter -($C_1$-$C_{12}$)-Alkyl, -($C_1$-$C_{12}$)-Alkoxy, -(C3-$C_{12}$)-Cycloalkyl, -($C_3$-$C_{12}$)-Heterocycloalkyl, -($C_6$-$C_{20}$)-Aryl, Fluor, Chlor, Cyano, Formyl, Acyl oder Alkoxycarbonyl. Substituierte -($C_3$-$C_{12}$)-Cycloalkylgruppen tragen vorzugsweise eine oder mehrere -($C_1$-$C_6$)-Alkylgruppen. Substituierte -($C_3$-$C_{12}$)-Heterocycloalkylgruppen tragen vorzugsweise eine oder mehrere -($C_1$-$C_6$)-Alkylgruppen.

Der Ausdruck -($C_6$-$C_{20}$)-Aryl umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -($C_6$-$C_{10}$)-Aryl und -($C_6$-$C_{10}$)-Aryl-($C_6$-$C_{10}$)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -($C_6$-$C_{20}$)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen (wie Cl, F, Br, I), -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3$H; -$SO_3$Na, - $NO_2$, -CN, -$NH_2$, -N[($C_1$-$C_{12}$)-Alkyl]$_2$.

Substituierte -($C_6$-$C_{20}$)-Arylgruppen sind vorzugsweise substituierte -($C_6$-$C_{10}$)-Arylgruppen und -($C_6$-$C_{10}$)-Aryl-($C_6$-$C_{10}$)-Arylgruppen, insbesondere substituiertes Phenyl oder substituiertes Naphthyl oder substituiertes Anthracenyl. Substituierte -($C_6$-$C_{20}$)-Arylgruppen tragen vorzugsweise eine oder mehrere z.B. 1, 2, 3, 4 oder 5 Substituenten, ausgewählt unter -($C_1$-$C_{12}$)-Alkylgruppen, -($C_1$-$C_{12}$)-Alkoxygruppen.

In einer Ausführungsform sind $R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ jeweils unabhängig voneinander ausgewählt aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl.

In einer Ausführungsform stehen $R^{41}$, $R^{43}$, $R^{51}$, $R^{53}$, $R^{61}$, $R^{63}$ jeweils für *tert*-Butyl.

In einer Ausführungsform stehen $R^{45}$, $R^{55}$, $R^{65}$ jeweils für -H.

In einer Ausführungsform ist das Gemisch bei 25 °C fest.
Der Begriff "fest" ist in diesem Zusammenhang als Aggregatzustand zu verstehen, womit sich das Gemisch beispielsweise von einem flüssigen Gemisch abgrenzt.

Neben dem Gemisch wird auch ein Komplexgemisch beansprucht, welcher ein solches Gemisch aufweist.

Komplexgemisch umfassend:

- ein zuvor beschriebenes Gemisch,
- ein Metallatom ausgewählt aus: Rh, Ru, Co, Ir.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Neben den Gemischen / Komplexgemischen wird auch deren Verwendung als Komplexgemische zur Katalyse einer Hydroformylierungsreaktion beansprucht. Hierbei stellen die Verbindungen des Gemisches die Liganden des Komplexes dar. Die Liganden koordinieren an das Metall-Zentralatom. Der so erhaltene Ligand-Metall-Komplex bzw. die so erhaltenen Komplexgemische katalysieren dann die Hydroformylierungsreaktion.

Verwendung eines zuvor beschriebenen Gemisches in einem Komplexgemisch zur Katalyse einer Hydroformylierungsreaktion.

Des Weiteren wird auch die Hydroformylierungsreaktion beansprucht.

Verfahren umfassend die Verfahrensschritte:

a) Vorlegen eines Olefins,
b) Zugabe eines Gemisches umfassend eine Verbindungen gemäß der Struktur **IIIb:**

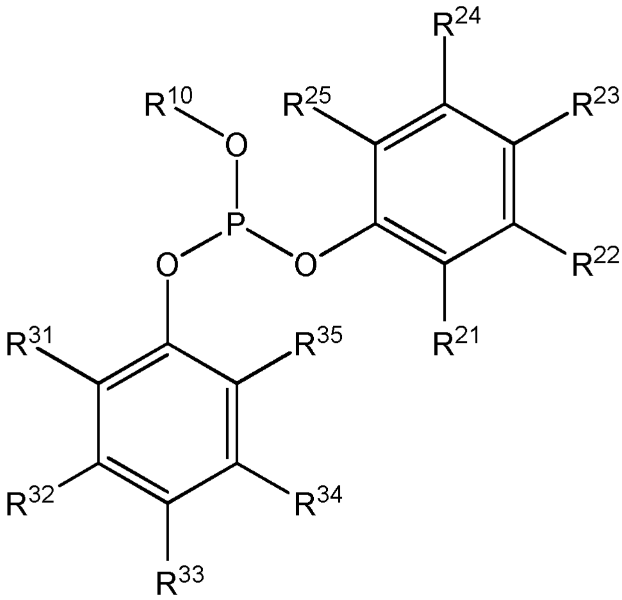

IIIb

wobei

$R^{10}$ ausgewählt ist aus:

$-(C_1-C_{12})$-Alkyl,

$R^{21}$, $R^{22}$, $R^{23}$, $R^{24}$, $R^{25}$, $R^{31}$, $R^{32}$, $R^{33}$, $R^{34}$, $R^{35}$ jeweils unabhängig voneinander ausgewählt sind aus: -H, $-(C_1-C_{12})$-Alkyl, $-O-(C_1-C_{12})$-Alkyl, $-O-(C_6-C_{20})$-Aryl, $-(C_6-C_{20})$-Aryl, -Halogen, $-COO-(C_1-C_{12})$-Alkyl, $-CO-(C_1-C_{12})$-Alkyl, $-CO-(C_6-C_{20})$-Aryl, -COOH, -OH;
sowie eine Verbindung gemäß der Struktur **IIb:**

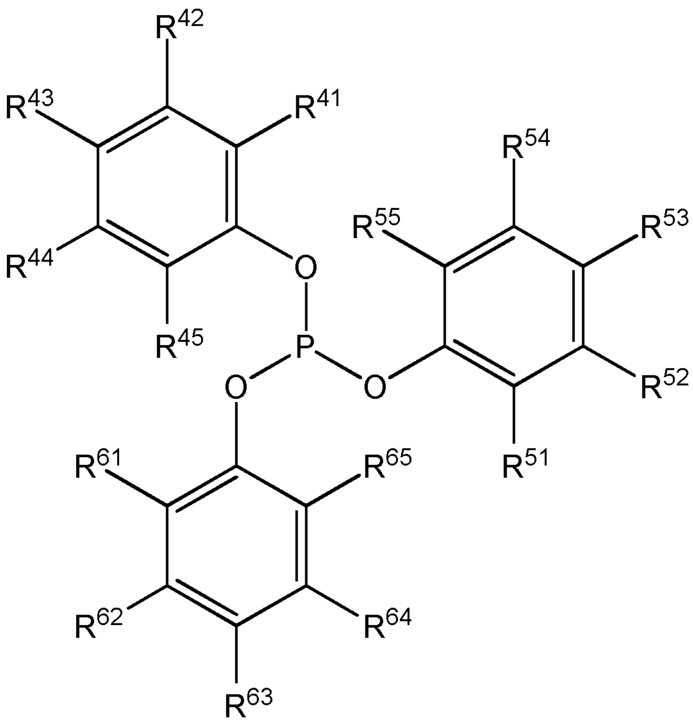

IIb

wobei

$R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ jeweils unabhängig voneinander ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CO-($C_1$-$C_{12}$)-Alkyl, -CO-($C_6$-$C_{20}$)-Aryl, -COOH, -OH;

c) Zugabe einer Verbindung welche eines der folgenden Metalle umfasst: Rh, Ru, Co, Ir,
d) Zuführen von $H_2$ und CO,
e) Erwärmen des Reaktionsgemisches, wobei das Olefin zu einem Aldehyd umgesetzt wird, wobei die Zugaben in den Verfahrensschritten b) und c) auch in einem Schritt durch die Zugabe eines entsprechenden Komplexgemisches erfolgen kann.

Hierbei können die Verfahrensschritte a) bis e) in beliebiger Reihenfolge erfolgen.
Bevorzugt sind eine Temperatur von 80 °C bis 160 °C und ein Druck von 1 bis 300 bar. Besonders bevorzugt sind eine Temperatur von 100 °C bis 160 °C und ein Druck von 15 bis 250 bar.
In einer Variante des Verfahrens stehen $R^{21}$, $R^{23}$, $R^{31}$, $R^{33}$ jeweils für *tert*-Butyl.
In einer Variante des Verfahrens stehen $R^{25}$, $R^{35}$ jeweils für $-CH_3$.
In einer Variante des Verfahrens steht $R^{10}$ für $-CH_2CH_3$.
In einer Variante des Verfahrens sind $R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$ $R^{64}$, $R^{65}$ jeweils unabhängig voneinander ausgewählt aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl.
In einer Variante des Verfahrens stehen $R^{41}$, $R^{43}$, $R^{51}$, $R^{53}$, $R^{61}$, $R^{63}$ jeweils für *tert*-Butyl.
In einer Variante des Verfahrens stehen $R^{45}$, $R^{55}$, $R^{65}$ jeweils für -H.
In einer bevorzugten Ausführungsform ist das Metall Rh.
Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende $C_6$-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende $C_8$-Olefingemisch (Di-n-buten, Di-iso-buten), Nonene, 2- oder 3-Methyloctene, das bei der Trimerisierung von Propen anfallende $C_9$-Olefingemisch (Tripropen), Decene, 2-Ethyl-1-octen, Dodecene, das bei der Tetramerisierung von Propen oder der Trimerisierung von Butenen anfallende $C_{12}$-Olefingemisch (Tetrapropen oder Tributen), Tetradecene, Hexadecene, das bei der Tetramerisierung von Butenen anfallende $C_{16}$-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.
Mit dem erfindungsgemäßen Verfahren können unter Verwendung der erfindungsgemäßen Gemische / Komplexgemische $\alpha$-Olefine, endständig verzweigte, innenständige und innenständig verzweigte Olefine hydroformyliert werden. Bemerkenswert ist dabei die hohe Ausbeute an endständig hydrofomyliertem Olefin, selbst wenn im Edukt nur ein geringer Anteil an Olefinen mit endständiger Doppelbindung vorhanden war.
Im Folgenden soll die Erfindung anhand von Ausführungsbeispielen näher erläutert werden.

**Allgemeine Arbeitsvorschriften**

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego, Christina Chai, Butterworth Heinemann (Elsevier), 6. Auflage, Oxford 2009).
Alle präparativen Arbeiten erfolgten in ausgeheizten Gefäßen. Die Charakterisierung der Produkte erfolgte mittels NMR-Spektroskopie. Chemische Verschiebungen ($\delta$) werden in ppm angegeben. Die Referenzierung der $^{31}$P-NMR-Signale erfolgte gemäß: $SR_{31P} = SR_{1H} * (BF_{31P} / BF_{1H}) = SR_{1H} * 0{,}4048$.
(Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Die Aufnahme von Kernresonanzspektren erfolgte mittels eines Bruker Avance 300 bzw. Bruker Avance 400, die gaschromatografische Analyse an Agilent GC 7890A.

Herstellung von Bis(2,4-di-tert.-butyl-6-methylphenyl)-ethylphosphit

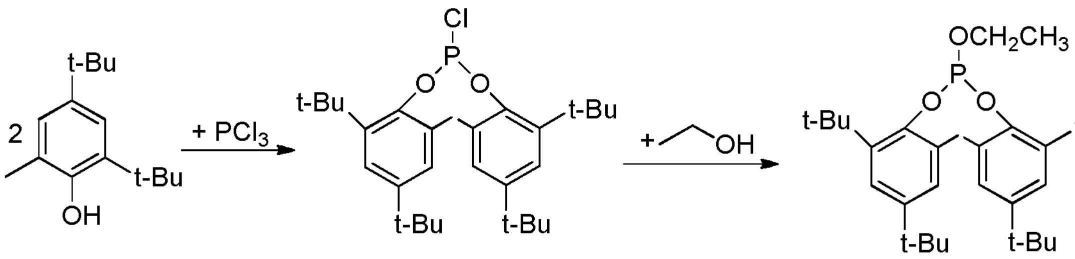


In einem 250 ml Schlenkkolben mit Magnetrührer, Aufsatz, Tropftrichter und Rückflusskühler wurden 22.5 g (0,1 mol) 2,4-Di-tert.-butyl-6-methylphenol (4,6-Di-tert-butyl-ortho-cresol) vorgelegt und zum Schmelzen des Phenols auf 55 °C erwärmt. Zur Schmelze wurden 0,13 ml (0,0015 mol) getrocknetes, entgastes Dimethylformamid zugegeben. Anschließend wurde innerhalb von 2 Stunden 5,7 ml (0,065 mol) Phosphortrichlorid zugetropft. Nach beendeter Zugabe wurde die Reaktionsmischung innerhalb 3 Stunden auf 140 °C erhitzt und 1 Stunden bei dieser Temperatur gerührt. Dann wurde unter Vakuum 1 Stunden bei 130 °C gerührt. Danach wurde die erhaltene klare gelb-orange Schmelze (=Bis(2,4-di-tert-butyl-6-methyl)-phosphorchloridit) über Nacht auf 80 °C abgekühlt und mit 75 ml entgastem Benzin (80-110 °C) verdünnt. Nach dem Abkühlen der Lösung auf - 5 °C wurden innerhalb von 15 Minuten 9.1 ml (0,0665 mol) entgastes Triethylamin zugegeben. Anschließend wurde innerhalb von 2 Stunden 4.4 mL (0,075 mol) getrocknetes und entgastes Ethanol zu getropft, wobei die Temperatur nicht über 5 °C anstieg. Dieses Gemisch wurde unter Rühren langsam über Nacht auf Raumtemperatur erwärmt. Am nächsten Morgen wurde das ausgefallene Triethylaminhydrochlorid abfiltriert und das Filtrat unter vermindertem Druck eingeengt. Es entstand ein weißer Rückstand, der in 60 mL entgastem Ethanol umkristallisiert wurde. Das Produkt konnte so in 73,9%iger (19,03g) Ausbeute als weißer Feststoff in 98%iger Reinheit laut LC-MS erhalten werden.

**Durchführung der Katalyseversuche**

Versuchsbeschreibung - allgemein

In einem 100 ml-Autoklaven der Fa. Parr Instruments wurde bei 120 °C, 50 bar Synthesegasdruck ($CO/H_2$ = 1:1 (Vol-%)) n-Octene hydroformyliert. Als Precursor wurden bei einer Katalysatorkonzentration von 100 ppm Rh bezogen auf das gesamte Reaktionsgemisch 0.123 g $Rh(acac)(CO)_2$ vorgelegt. Als Lösemittel wurden jeweils 40 bis 46 g Toluol verwendet. Der Ligand 1 bzw. der Ligand 2 bzw. die Ligandenmischung bestehend aus den Liganden 1 und 2 wurde in unterschiedlichen molaren Überschüssen relativ zum Rhodium verwendet. Zusätzlich wurden als GC-Standard ca. 0.5 g Tetraisopropylbenzol (TIPB) zugefügt. Ca. 6 g Edukt wurden nach Erreichen der vorgesehenen Reaktionstemperatur zudosiert.
Während der Reaktion wurde der Druck über eine Synthesegasregelung mit Massedurchflussmesser konstant gehalten. Die Rührerdrehzahl betrug 1200 $min^{-1}$. Proben wurden aus der Reaktionsmischung nach 12 Stunden gezogen. Die Ergebnisse der Versuche sind in Tabelle 1 zusammengefasst. (acac = acetylacetonat)

In den Katalyseversuchen verwendete Liganden:

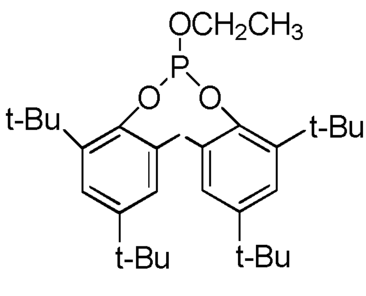

**1**

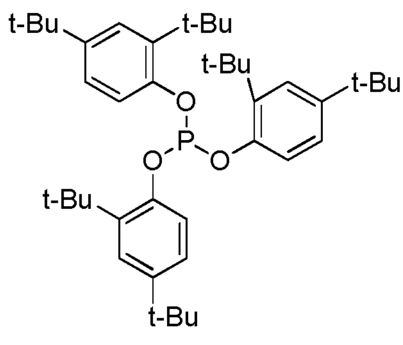

**2**

Die Herstellung des Liganden 1 ist im obigen experimentellen Teil beschreiben. Der Ligand 2 (TDTBPP bzw. Alkanox 240) ist kommerziell erhältlich.

In Tabelle 1 sind die Ergebnisse für die Hydroformylierung von Di-n-Buten angegeben. Di-n-Buten ist ein Gemisch aus Isomeren von n-Octenen (ca. 16 %), 3-Methyl-heptenen (ca. 65 %) und 3,4-Dimethylhexenen (ca. 19 %).

(Ausbeute = Aldehyd und Alkohol Gesamtausbeute; S = n/iso-Selektivität der im Gemisch enthaltenen Octene zum linearen Produkt)

Tabelle 1:

| Eintrag | Ligand A | Ligand B | Ligand A in [mmol] | Ligand B in [mmol] | A in % | S in % |
|---|---|---|---|---|---|---|
| 1 | - | 2 | - | 20** | 96,9 | 21,8 |
| 2* | 1 | 2 | 0,62 | 0,32 | 95,9 | 36,4 |
| 3* | 1 | 2 | 0,47 | 0,47 | 95,7 | 24,6 |
| 4* | 1 | 2 | 0,31 | 0,62 | 95,5 | 23,4 |
| Reaktionsbedingungen: 50 bar Synthesegasdruck, T = 140 °C, Substrat: Di-n-buten, P : Rh = 20 : 1; 100 ppm [Rh], 12 Stunden<br>* erfindungsgemäße Gemisch bzw. Komplexgemische<br>** Verhältnis Ligand zu Rh 20:1 | | | | | | |

Tabelle 1 enthält Versuche zur Hydroformylierung von Di-n-buten mit verschiedenen Gemischen / Komplexgemischen. Eintrag 1 zeigt einen Vergleichsversuch, welcher nur mit dem Ligand 2 durchgeführt wurde. Hier wurde zwar eine gute Ausbeute erzielt, jedoch lässt die Selektivität zu wünschen übrig.

Durch den Einsatz der erfindungsgemäßen Gemische / Komplexgemische konnte die Selektivität in allen Fällen gesteigert werden. Die Selektivität zu den gewünschten linearen Aldehyden ist hierbei merklich größer als bei dem kommerziell verfügbaren Liganden 2. In den Ausbeuten ist keine signifikante Verschlechterung zu erkennen.

Durch die Verwendung der erfindungsgemäßen Gemische / Komplexgemische, ist es möglich den Anteil an endständig hydroformylierten Produkt selektiv zu steuern und zu steigern.

Mit dem erfindungsgemäßen Verfahren können unter Verwendung der erfindungsgemäßen Gemische / Komplexgemische $\alpha$-Olefine, endständig verzweigte, innenständige und innenständig verzweigte Olefine hydroformyliert werden. Bemerkenswert ist dabei die hohe Ausbeute an endständig hydrofomyliertem Olefin, selbst wenn im Edukt nur ein geringer Anteil an Olefinen mit endständiger Doppelbindung vorhanden war.

Somit konnte mit Hilfe der obigen Beispiele gezeigt werden, dass die gestellten Aufgaben durch den Einsatz der erfindungsgemäßen Gemische / Komplexgemische gelöst wurden.

## Patentansprüche

**1.** Gemisch umfassend eine Verbindungen gemäß der Strukturen **IIIb:**

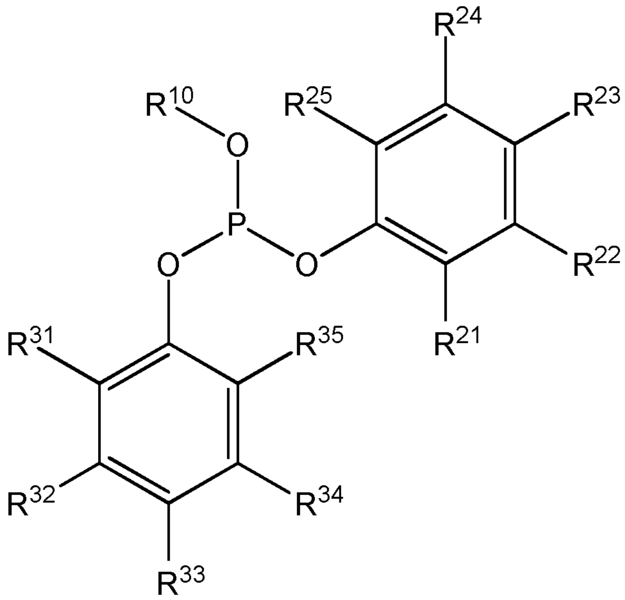

IIIb

wobei

$R^{10}$ ausgewählt ist aus:

$-(C_1-C_{12})$-Alkyl,

$R^{21}$, $R^{22}$, $R^{23}$, $R^{24}$, $R^{25}$, $R^{31}$, $R^{32}$, $R^{33}$, $R^{34}$, $R^{35}$ jeweils unabhängig voneinander ausgewählt sind aus: -H, $-(C_1-C_{12})$-Alkyl, $-O-(C_1-C_{12})$-Alkyl, $-O-(C_6-C_{20})$-Aryl, $-(C_6-C_{20})$-Aryl, -Halogen, $-COO-(C_1-C_{12})$-Alkyl, $-CO-(C_1-C_{12})$-Alkyl, $-CO-(C_6-C_{20})$-Aryl, -COOH, -OH;
sowie
umfassend eine Verbindung gemäß der Struktur **IIb:**

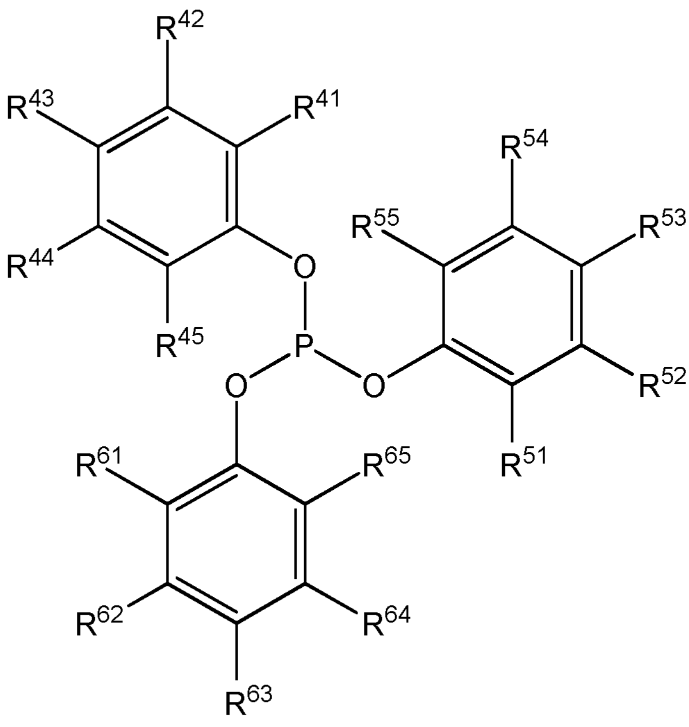

IIb

wobei

$R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ jeweils unabhängig voneinander ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CO-($C_1$-$C_{12}$)-Alkyl, -CO-($C_6$-$C_{20}$)-Aryl, -COOH, -OH.

**2.** Gemisch nach Anspruch 1,
wobei $R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ jeweils unabhängig voneinander ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl.

**3.** Gemisch nach einem der Ansprüche 1 bis 2,
wobei $R^{41}$, $R^{43}$, $R^{51}$, $R^{53}$, $R^{61}$, $R^{63}$ jeweils für *tert*-Butyl stehen.

**4.** Komplexgemisch umfassend:

- ein Gemisch nach einem der Ansprüche 1 bis 3,
- ein Metallatom ausgewählt aus: Rh, Ru, Co, Ir.

**5.** Verwendung eines Gemisches nach einem der Ansprüche 1 bis 3,
in einem Komplexgemisch zur Katalyse einer Hydroformylierungsreaktion.

**6.** Verfahren umfassend die Verfahrensschritte:

a) Vorlegen eines Olefins,
b) Zugabe eines Gemisches umfassend eine Verbindungen gemäß der Struktur **IIIb:**

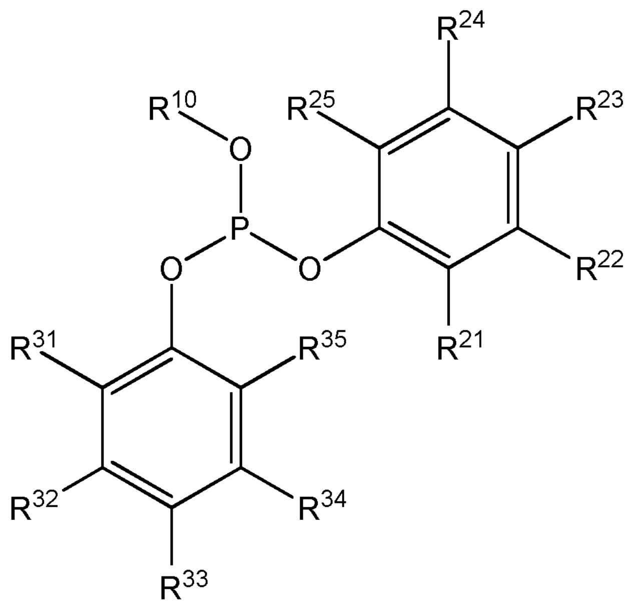

**IIIb**

wobei

$R^{10}$ ausgewählt ist aus:

-($C_1$-$C_{12}$)-Alkyl,

$R^{21}$, $R^{22}$, $R^{23}$, $R^{24}$, $R^{25}$, $R^{31}$, $R^{32}$, $R^{33}$, $R^{34}$, $R^{35}$ jeweils unabhängig voneinander ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CO-($C_1$-$C_{12}$)-Alkyl, -CO-($C_6$-$C_{20}$)-Aryl, -COOH, -OH;
sowie eine Verbindung gemäß der Struktur **IIb:**

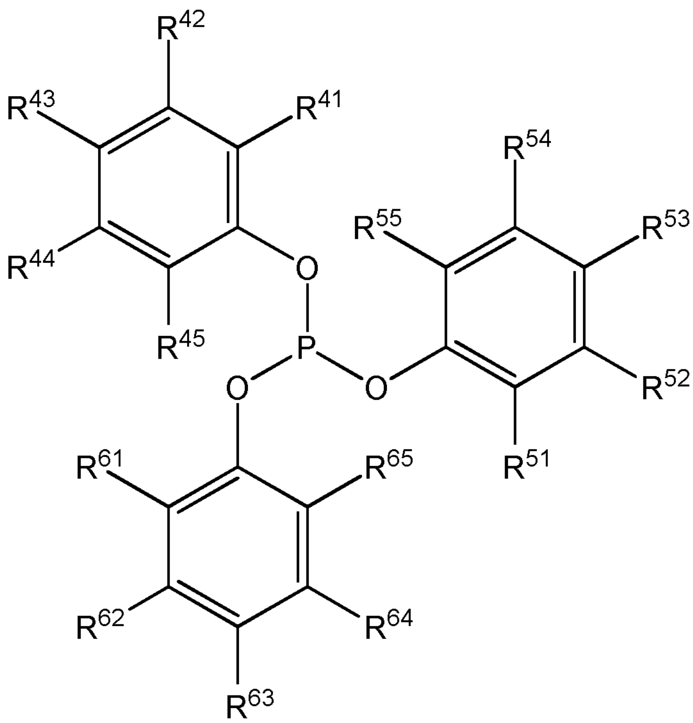

**IIb**

wobei

$R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ jeweils unabhängig voneinander ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CO-($C_1$-$C_{12}$)-Alkyl, -CO-($C_6$-$C_{20}$)-Aryl, -COOH, -OH;

c) Zugabe einer Verbindung welche eines der folgenden Metalle umfasst: Rh, Ru, Co, Ir,
d) Zuführen von $H_2$ und CO,
e) Erwärmen des Reaktionsgemisches, wobei das Olefin zu einem Aldehyd umgesetzt wird, wobei die Zugaben in den Verfahrensschritten b) und c) auch in einem Schritt durch die Zugabe eines entsprechenden Komplexgemisches erfolgen kann.

**7.** Verfahren nach Anspruch 6,
wobei $R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ jeweils unabhängig voneinander ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl.

**8.** Verfahren nach einem der Ansprüche 6 oder 7,
wobei $R^{41}$, $R^{43}$, $R^{51}$, $R^{53}$, $R^{61}$, $R^{63}$ jeweils für *tert*-Butyl stehen.

## Claims

**1.** Mixture comprising a compound of the structure **IIIb:**

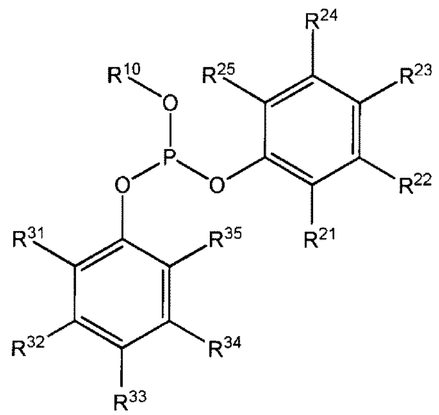

**IIIb**

where

$R^{10}$ is selected from:

-($C_1$-$C_{12}$)-alkyl,

$R^{21}$, $R^{22}$, $R^{23}$, $R^{24}$, $R^{25}$, $R^{31}$, $R^{32}$, $R^{33}$, $R^{34}$, $R^{35}$ are each independently selected from: -H, - ($C_1$-$C_{12}$)-alkyl, -O-($C_1$-$C_{12}$) -alkyl, -O-($C_6$-$C_{20}$)-aryl, - ($C_6$-$C_{20}$)-aryl, -halogen, -COO-($C_1$-$C_{12}$)-alkyl, -CO-($C_1$-$C_{12}$)-alkyl, -CO-($C_6$-$C_{20}$)-aryl, -COOH, -OH;
and
comprising a compound of the structure **IIb:**

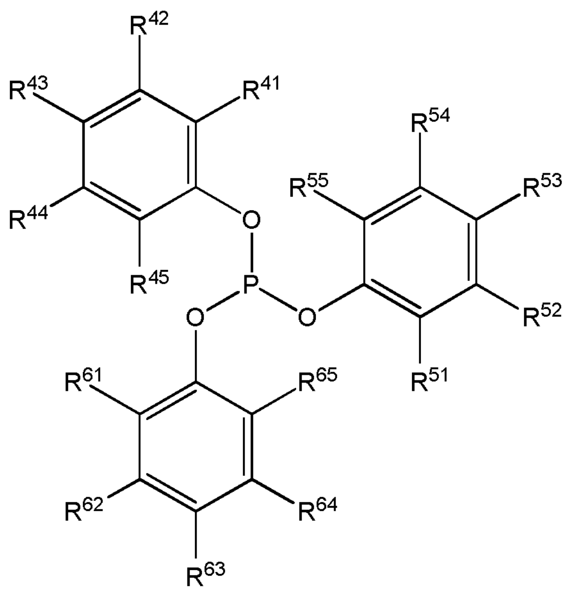

**IIb**

where

$R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ are each independently selected from: -H, -($C_1$-$C_{12}$)-alkyl, -O-($C_1$-$C_{12}$)-alkyl, -O-($C_6$-$C_{20}$)-aryl, -($C_6$-$C_{20}$)-aryl, -halogen, -COO-($C_1$-$C_{12}$)-alkyl, -CO-($C_1$-$C_{12}$)-alkyl, -CO-($C_6$-$C_{20}$)-aryl, -COOH, -OH.

**2.** Mixture according to Claim 1,
where $R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ are each independently selected from: -H, -($C_1$-$C_{12}$)-alkyl, -O-($C_1$-$C_{12}$)-alkyl, -O-($C_6$-$C_{20}$)-aryl, -($C_6$-$C_{20}$)-aryl.

**3.** Mixture according to either of Claims 1 and 2, where $R^{41}$, $R^{43}$, $R^{51}$, $R^{53}$, $R^{61}$, $R^{63}$ are each tert-butyl.

**4.** Complex mixture comprising:

- a mixture according to any of Claims 1 to 3,
- a metal atom selected from: Rh, Ru, Co, Ir.

**5.** Use of a mixture according to any of Claims 1 to 3 in a complex mixture for catalysis of a hydroformylation reaction.

**6.** Process comprising the process steps of:

a) initially charging an olefin,
b) adding a mixture comprising a compound of the structure **IIIb:**

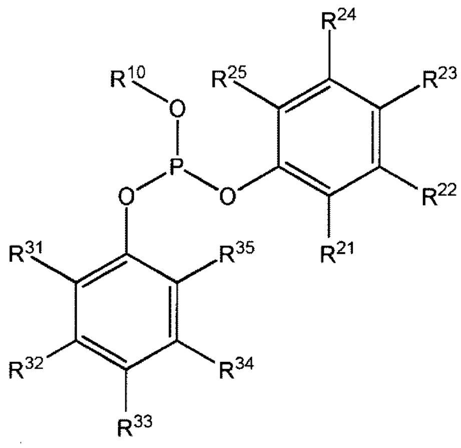

**IIIb**

where

$R^{10}$ is selected from:

- $(C_1$-$C_{12})$ -alkyl,

$R^{21}$, $R^{22}$, $R^{23}$, $R^{24}$, $R^{25}$, $R^{31}$, $R^{32}$, $R^{33}$, $R^{34}$, $R^{35}$ are each independently selected from: -H, -$(C_1$-$C_{12})$-alkyl, -O-$(C_1$-$C_{12})$-alkyl, -O-$(C_6$-$C_{20})$-aryl, -$(C_6$-$C_{20})$-aryl, -halogen, -COO-$(C_1$-$C_{12})$-alkyl, -CO-$(C_1$-$C_{12})$-alkyl, -CO-$(C_6$-$C_{20})$-aryl, -COOH, -OH;
and a compound of the structure **IIb:**

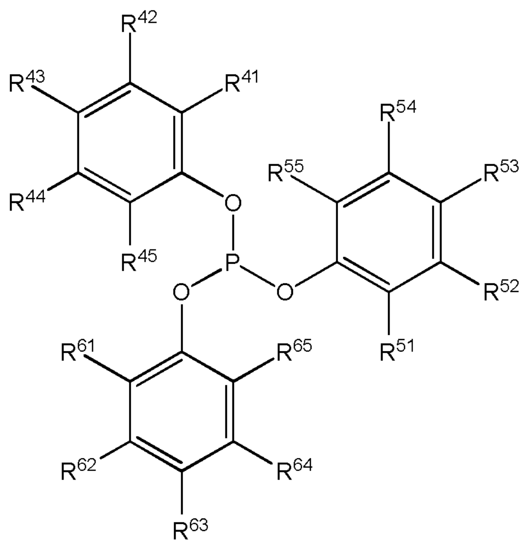

**IIb**

where

$R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ are each independently selected from: -H, -($C_1$-$C_{12}$)-alkyl, -O-($C_1$-$C_{12}$)-alkyl, -O-($C_6$-$C_{20}$)-aryl, -($C_6$-$C_{20}$)-aryl, -halogen, -COO-($C_1$-$C_{12}$)-alkyl, -CO-($C_1$-$C_{12}$)-alkyl, -CO-($C_6$-$C_{20}$)-aryl, -COOH, -OH;

c) adding a compound comprising one of the following metals: Rh, Ru, Co, Ir,
c) feeding in $H_2$ and CO,
d) heating the reaction mixture, with conversion of the olefin to an aldehyde,

where the additions in process steps b) and c) can also be effected in one step through the addition of a corresponding complex mixture.

**7.** Process according to Claim 6, where $R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ are each independently selected from: -H, -($C_1$-$C_{12}$)-alkyl, -O-($C_1$-$C_{12}$)-alkyl, -O-($C_6$-$C_{20}$)-aryl, -($C_6$-$C_{20}$)-aryl.

**8.** Process according to either of Claims 6 and 7, where $R^{41}$, $R^{43}$, $R^{51}$, $R^{53}$, $R^{61}$, $R^{63}$ are each tert-butyl.

**Revendications**

**1.** Mélange, comprenant un composé selon la structure IIIb :

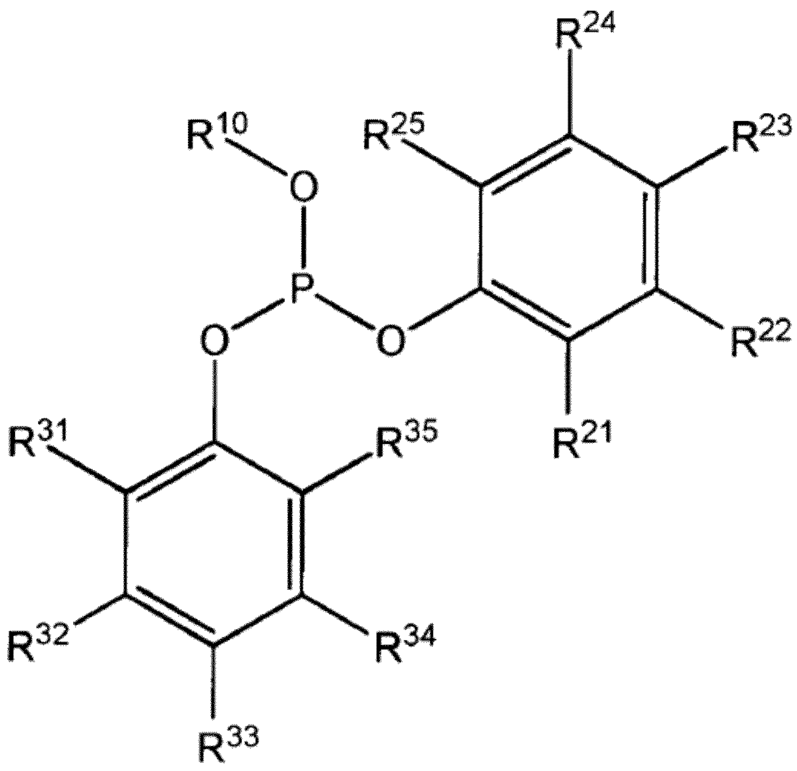

IIIb

dans laquelle

$R^{10}$ est choisi parmi :

alkyle en ($C_1$-$C_{12}$),

$R^{21}$, $R^{22}$, $R^{23}$, $R^{24}$, $R^{25}$, $R^{31}$, $R^{32}$, $R^{33}$, $R^{34}$, $R^{35}$ sont chacun choisis indépendamment les uns des autres parmi : -H, alkyle en ($C_1$-$C_{12}$), -O-alkyle en ($C_1$-$C_{12}$), -O-aryle en ($C_6$-$C_{20}$), aryle en ($C_6$-$C_{20}$), halogène, -COO-alkyle en ($C_1$-$C_{12}$), -CO-alkyle en ($C_1$-$C_{12}$), -CO-aryle en ($C_6$-$C_{20}$), -COOH, -OH ;
et
comprenant un composé selon la structure IIb :

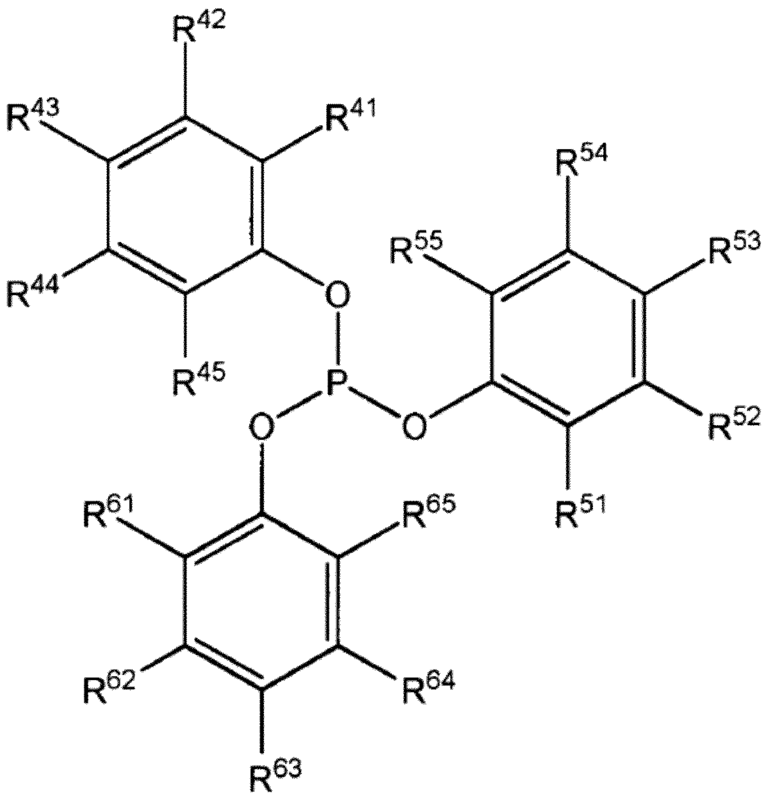

IIb

dans laquelle

$R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ sont chacun choisis indépendamment les uns des autres parmi :
-H, alkyle en ($C_1$-$C_{12}$), -O-alkyle en ($C_1$-$C_{12}$), -O-aryle en ($C_6$-$C_{20}$), aryle en ($C_6$-$C_{20}$), halogène, -COO-alkyle en ($C_1$-$C_{12}$), -CO-alkyle en ($C_1$-$C_{12}$), -CO-aryle en ($C_6$-$C_{20}$), -COOH, -OH.

**2.** Mélange selon la revendication 1, dans lequel $R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ sont chacun choisis indépendamment les uns des autres parmi :
-H, alkyle en ($C_1$-$C_{12}$), -O-alkyle en ($C_1$-$C_{12}$), -O-aryle en ($C_6$-$C_{20}$), aryle en ($C_6$-$C_{20}$).

**3.** Mélange selon l'une quelconque des revendications 1 à 2, dans lequel $R^{41}$, $R^{43}$, $R^{51}$, $R^{53}$, $R^{61}$, $R^{63}$ représentent chacun tert-butyle.

**4.** Mélange complexe, comprenant :

- un mélange selon l'une quelconque des revendications 1 à 3,
- un atome métallique choisi parmi : Rh, Ru, Co, Ir.

**5.** Utilisation d'un mélange selon l'une quelconque des revendications 1 à 3 dans un mélange complexe pour la catalyse d'une réaction d'hydroformylation.

**6.** Procédé comprenant les étapes de procédé suivantes :

a) le chargement d'une oléfine,
b) l'ajout d'un mélange comprenant un composé selon la structure IIIb :

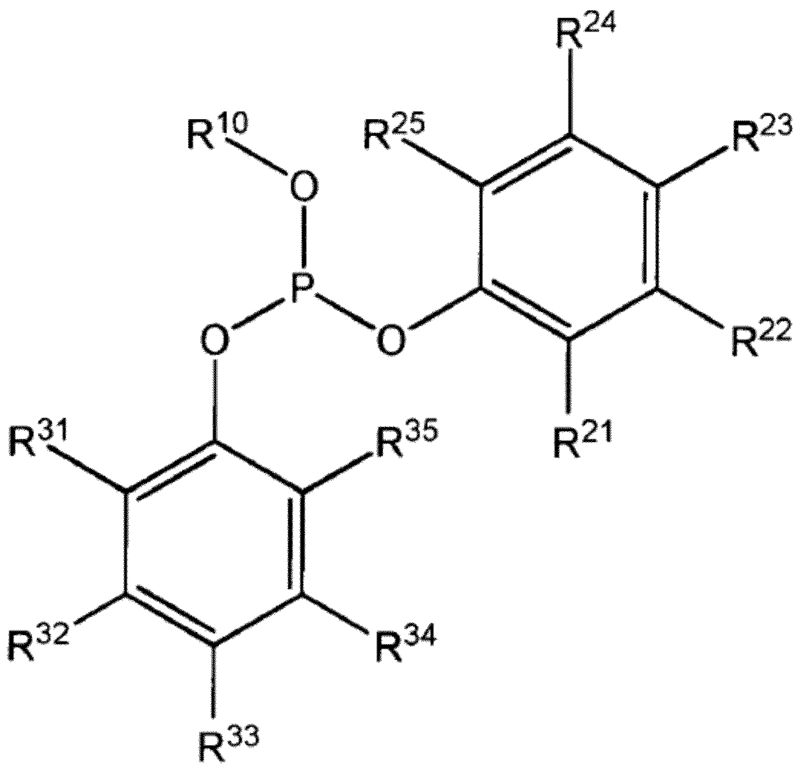

IIIb

dans laquelle

$R^{10}$ est choisi parmi :

alkyle en ($C_1$-$C_{12}$),

$R^{21}$, $R^{22}$, $R^{23}$, $R^{24}$, $R^{25}$, $R^{31}$, $R^{32}$, $R^{33}$, $R^{34}$, $R^{35}$ sont chacun choisis indépendamment les uns des autres parmi :
-H, alkyle en ($C_1$-$C_{12}$), -O-alkyle en ($C_1$-$C_{12}$), -O-aryle en ($C_6$-$C_{20}$), aryle en ($C_6$-$C_{20}$), halogène, -COO-alkyle en ($C_1$-$C_{12}$), -CO-alkyle en ($C_1$-$C_{12}$), -CO-aryle en ($C_6$-$C_{20}$), -COOH, -OH ;
et un composé selon la structure IIb :

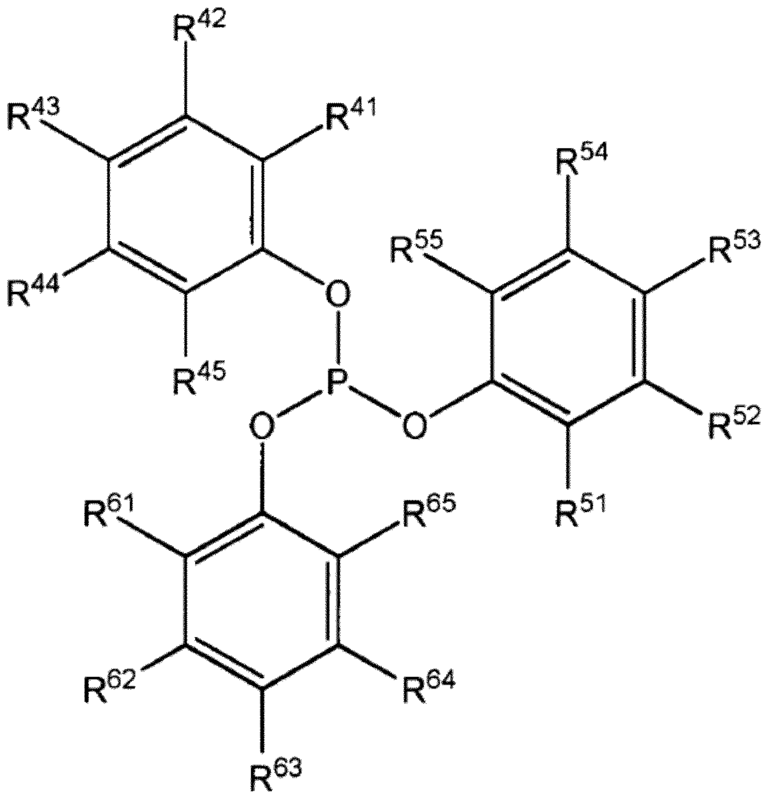

IIb

dans laquelle

$R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ sont chacun choisis indépendamment les uns des autres parmi :
-H, alkyle en ($C_1$-$C_{12}$), -O-alkyle en ($C_1$-$C_{12}$), -O-aryle en ($C_6$-$C_{20}$), aryle en ($C_6$-$C_{20}$), halogène, -COO-alkyle en ($C_1$-$C_{12}$), -CO-alkyle en ($C_1$-$C_{12}$), -CO-aryle en ($C_6$-$C_{20}$), -COOH, -OH ;

c) l'ajout d'un composé qui comprend un des métaux suivants : Rh, Ru, Co, Ir,
d) l'introduction d'$H_2$ et de CO,
e) le chauffage du mélange réactionnel, l'oléfine étant transformée en un aldéhyde,

les ajouts aux étapes de procédé b) et c) pouvant également avoir lieu en une étape par l'ajout d'un mélange complexe correspondant.

**7.** Procédé selon la revendication 6, dans lequel $R^{41}$, $R^{42}$, $R^{43}$, $R^{44}$, $R^{45}$, $R^{51}$, $R^{52}$, $R^{53}$, $R^{54}$, $R^{55}$, $R^{61}$, $R^{62}$, $R^{63}$, $R^{64}$, $R^{65}$ sont chacun choisis indépendamment les uns des autres parmi :
-H, alkyle en ($C_1$-$C_{12}$), -O-alkyle en ($C_1$-$C_{12}$), -O-aryle en ($C_6$-$C_{20}$), aryle en ($C_6$-$C_{20}$).

**8.** Procédé selon l'une quelconque des revendications 6 ou 7, dans lequel $R^{41}$, $R^{43}$, $R^{51}$, $R^{53}$, $R^{61}$, $R^{63}$ représentent chacun tert-butyle.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20120253080 A
- EP 1099678 A
- WO 2007149143 A
- US 8258215 B2


### In der Beschreibung aufgeführte Nicht-Patentliteratur


- **B. CORNILS ; W. A. HERRMANN.** Applied Homogeneous Catalysis with Organometallic Compounds. VCH, 1996, vol. 1 & 2
- **R. FRANKE ; D. SELENT ; A. BÖRNER.** Applied Hydroformylation. *Chem. Rev.,* 2012
- **B. L. HAYMORE ; A. VAN HASSELT ; R. BECK.** *Annals of the New York Acad. Sci.,* 1983, vol. 415, 159-175
- **P. W. N. M. VAN LEEUWEN.** Rhodium Catalyzed Hydroformylation. Kluwer, 2000
- **KLAUS-DIETHER WIESE.** Dietmar Obst. *Top. Organomet. Chem.,* 2006, vol. 18, 1-33
- **BÖRNER.** *Angew. Chem. Int. Ed.,* 2000, vol. 39 (9), 1639-1641
- **VAN LEEUWEN.** *Journal of Catalysis,* 2013, vol. 298, 198-205
- **MANFRED T. REETZ et al.** The Influence of Mixtures of Monodentate Achiral Ligands on the Regioselectivity of Transition-Metal-Catalyzed Hydroformylation. *ANGEWANDTE CHEMIE INTERNATIONAL EDITION,* 06. Mai 2005, vol. 44 (19), 2962-2964
- **R. FRANKE ; D. SELENT ; A. BÖRNER.** Applied Hydroformylation. *Chem. Rev.,* 2012, 5688
- **P. W. N. M. VAN LEEUWEN.** Rhodium Catalyzed Hydroformylation. Kluwer, 2000, 48 ff, , 233 ff
- **K.D. WIESE ; D. OBST.** Top. Organomet. Chem. Springer Verlag, 2006, vol. 18, 1-13
- **W. L. F. ARMAREGO ; CHRISTINA CHAI.** Purification of Laboratory Chemicals. Butterworth Heinemann (Elsevier), 2009
- **ROBIN K. HARRIS ; EDWIN D. BECKER ; SONIA M. CABRAL DE MENEZES ; ROBIN GOODFELLOW ; PIERRE GRANGER.** *Pure Appl. Chem.,* 2001, vol. 73, 1795-1818
- **ROBIN K. HARRIS ; EDWIN D. BECKER ; SONIA M. CABRAL DE MENEZES ; PIERRE GRANGER ; ROY E. HOFFMAN ; KURT W. ZILM.** *Pure Appl. Chem.,* 2008, vol. 80, 59-84